# EUROPEAN PATENT SPECIFICATION

(11) **EP 2 863 932 B1**
(45) Date of publication and mention of the grant of the patent: **01.11.2017**
(21) Application number: 13729714.9
(22) Date of filing: 18.06.2013
(51) Int. Cl.: A61K 36/185, A61K 36/50, A61K 36/80, A61K 36/515, A61K 36/11, A61K 36/31, A61K 36/15, A61K 36/56, A61K 36/30, A61K 36/52, A61K 36/90, A61K 36/808, A61P 7/10

(54) **COMPOSITION FOR USE IN THE TREATMENT OF LYMPHEDEMA**
ZUSAMMENSETZUNG ZUR VERWENDUNG BEI DER BEHANDLUNG VON LYMPHÖDEMEN
COMPOSITION POUR SON UTILISATION DANS LE TRAITEMENT DU LYMPHOEDÈME

(30) Priority: 22.06.2012 EP 12173196
(43) Date of publication of application: 29.04.2015
(73) Proprietor: Biologische Heilmittel Heel GmbH, 76532 Baden-Baden (DE)
(72) Inventor: BURMEISTER, Yvonne, 76530 Baden-Baden (DE); SEILHEIMER, Bernd, 76530 Baden-Baden (DE)
(74) Representative: Herzog, Fiesser & Partner Patentanwälte PartG mbB
(86) International application number: PCT/EP2013/062569
(87) International publication number: WO 2013/189906

(56) References cited:
- anonymous: "Lamphomyosot: a dubious product", , 4 May 2003 (2003-05-04), XP002685757, Retrieved from the Internet: URL:http://www.homeowatch.org/reg/BHI/lymp homyosot.html [retrieved on 2012-10-22]
- anonymous: "Lymphomysot", Drugs.com , April 2010 (2010-04), XP002686083, Retrieved from the Internet: URL:http://www.drugs.com/pro/lymphomyosot. html?printable=1 [retrieved on 2012-10-23]
- Biologische Heilmittel Heel GmbH: "Lymphomyosot", , April 2001 (2001-04), XP002686084, Retrieved from the Internet: URL:http://ebookbrowse.com/gdoc.php?id=606 94830&url=902dc8a5c74f2e47d22444c220b06d85 [retrieved on 2012-10-22]
- ST.ZENNER, H. METELMANN: "Therapeutic use of lymphomyosot-Results of a multicentre use observation study ion 3512 patients", JOURNAL OF NATURAL MEDICINE, vol. 3, no. 3, June 1990 (1990-06), pages 1-19, XP002686085,
- anonymous: "Pre-dental visit liquid", Daily Med , 9 November 2011 (2011-11-09), pages 1-3, XP002685755, Retrieved from the Internet: URL:http://dailymed.nlm.nih.gov/dailymed/l ookup.cfm?setid=c6c60fda-203c-4da6-9695-61 c88e027c2f [retrieved on 2012-10-23]
- ANONYMOUS ED - ANONYMOUS: "Homöopathisches Arzneibuch1", 25 October 1985 (1985-10-25), HOMÖOPATHISCHES ARZNEIBUCH, DEUTSCHER APOTHEKERVERLAG, STUTTGART, PAGE(S) 1 - 12, XP002660266, ISBN: 3-7692-0932-X the whole document
- Harlan: "BALB/c", www.harlaneurope.com , 26 December 2005 (2005-12-26), pages 1-20, Retrieved from the Internet: URL:http://harlanisrael.com/blabc.pdf [retrieved on 2016-08-16]

## Description

The present invention is defined in claims 1 to 5.

Edema is swelling from excessive accumulation of fluid in the interstitial space of tissues or organs (Cho S, Atwood JE. Peripheral edema. The American Journal of Medicine. 2002;113(7):580-586.; Revis DR. Lymphedema. eMedicine 2010; http://emedicine.medscape.com/article/191350-overview Accessed October 8, 2010.). There are many different mechanisms that can increase the risk of developing edema including impaired circulation and removal of lymphatic fluid. Edema might only affect specific tissues and organs or be more generalized and widespread depending on the extent and duration of the pathology (Cho S, Atwood JE. Peripheral edema. The American Journal of Medicine. 2002;113(7):580-586.). The most common sites of edema including lymphedema are usually the feet and legs but it can also occur around other places including the eyes, neck and head region (Cho S, Atwood JE. Peripheral edema. The American Journal of Medicine. 2002;113(7):580-586.). The presence of varicose veins (from chronic venous insufficiency) and thrombophlebitis are two of the most common causes of a localized edema whereas more systemic causes of edema include lymphedema and hypothyroidism as well as an idiopathic type oedema also known as cyclical oedema or fluid retention syndrome (Revis DR. Lymphedema. eMedicine 2010; http://emedicine.medscape.com/article/191350-overview Accessed October 8, 2010.).

Lymphedema refers to the buildup of fluid, proteins and lipid in the interstitial space following some type of lymphatic system dysfunction (Revis DR. Lymphedema. eMedicine 2010; http://emedicine.medscape.com/article/191350-overview Accessed October 8, 2010.). The lymph vessels are responsible for the collection and transportation of the lymphatic fluid containing these high molecular weight proteins to the lymph nodes where it is filtered from waste and foreign materials. Any abnormality to this system such as congenital malformations found in primary lymphedema or blockage or injury to the lymphatic vessels of secondary lymphedema can lead to the accumulation of fluid resulting in abnormal swelling, tightness of skin and infection. Left untreated, lymphedema can progress to fibrosis, pain/paresthesias, extreme disfigurement, reduced immune responsiveness, and although rare, the development of lymphangiosarcoma.

Secondary lymphedema is the most common type of lymphedema with identifiable causes that destroys or renders inadequate the otherwise normal lymphatic system. Worldwide, the most common cause of secondary lymphedema is filariasis, the direct infestation of lymph nodes by the parasite *Wuchereria bancrofti* whereas removal of axillary lymph nodes as a result of breast cancer surgery is the most common cause in industrialized nations such as the USA (Revis DR. Lymphedema. eMedicine 2010; http://emedicine.medscape.com/article/191350-overview Accessed October 8, 2010.; Brennan MJ. Lymphedema following the surgical treatment of breast cancer: a review of pathophysiology and treatment. Journal of Pain and Symptom Management. 1992;7(2):110-116.). Melanoma, cervical, endometrial, vulvar, prostate, penile, and soft tissue cancers may also be associated with lymphedema in both upper and lower extremities in addition to other areas of the body (Maus EA, Tan IC, Rasmussen JC, Marshall MV, Fife CE, Smith LA, Guilliod R, Sevick-Muraca EM. Near-infrared fluorescence imaging of lymphatics in head and neck lymphedema. Head Neck. 2010 Nov 12.). Non-cancer related aetiologies of secondary lymphedema include infections such as cellulitis commonly found in obese patients, inflammatory and thrombotic type injuries caused by certain drugs, vein stripping, peripheral vascular surgery and deep vein thrombosis together with scarring caused by severe radiation therapy, bums and burn scar excision (Revis DR. Lymphedema. eMedicine 2010; http://emedicine.medscape.com/article/191350-overview Accessed October 8, 2010.; http://www.mayoclinic.com/health/lymphedema/DS00609.).

Studies estimate that anywhere from 30-60% of breast cancer survivors report symptoms of lymphedema with both mastectomy and conservative surgery patients reporting similar rates (Paskett 2000, Brest J 6: 373-8). During breast cancer surgery, the lymphatic vessels emanating from the arm are excised at the axilla along with their associated lymph nodes and surrounding connective tissue in an effort to prevent tumor metastasis through the lymphatic system and remove tumorous cells residing within the lymphatic system. The ensuing fibrotic wound repair process fills the surgical space with obstructive scar tissue, which has been found to reduce recovery of lymphatic drainage (Suami 2007, Lymphology 40: 122-6). Lymphedema usually develops several years after the original lymphatic-related injury has healed. It has been hypothesized that poor lymphatic regeneration across the obstructive scar tissue during the wound healing process may predispose the tissue to swell at a later date (Ongstad 2010, Am J Physiol Heart Circ Physiol 299: 46-54).

Lymphedema often causes cancer survivors to experience severe limitations in their daily activities, measurably affecting their overall quality of life. Specifically, women with lymphedema are more disabled, report symptoms from the upper extremity (shoulder or arm pain) and difficulties in arm movement, pain and swelling from the operated breast, and higher psychological distress (Chachaj 2010, Psychooncology 19: 299-305). Despite recent improvements in surgical techniques, such as the use of sentinel node biopsy for staging, as opposed to full axillary node dissection, the risk of lymphedema has declined but has not been eliminated (Leidenius 2005, J Surg Oncol 92: 23-31).

Currently there is no cure for lymphedema. There are subsequent non-curative treatments, such as compression garments and manual lymphatic drainage therapy, that are administered after the onset of lymphedema (Hamner 2007, Ann Surg Oncol 14: 1904-8). Lymphedema can be a long-term, uncomfortable, and painful condition requiring daily treatment. If not treated protein-rich fluid continues to accumulate in the tissue which can lead to fibrosis and reoccurring infections. Because fluid drainage in the human may be compromised by the failure of lymphatic vessels to regenerate during normal wound repair due to the abundant deposition of fibrotic tissue (Ongstad 2010, loc cit; Avraham 2009, Plastic and Reconstractive Surgery 124:438-50; Clavin 2008, Am J Physiol Heart Circ Physiol 295: 2113-27; Uzarski 2008, Am J Physiol Heart Circ Physiol 294: 1326-34), it is possible that improvements to the natural healing of the lymphatic-related injury may increase fluid drainage.

Homeopathic drugs have been shown in several studies to act on inflammatory pathways and reduce tissue edema (Macedo 2004, Homeopathy 93:84-87; Prado Neto 2004, Homeopathy 93:12-16). Lymphomyosot® or Lymphomyosot N® is a homeopathic agent that was suggested to exert some effects in the treatment of edemas of thrombotic or inflammatory aetiology. However, the mechanism of action and the effectiveness of Lymphomyosot® or Lymphomyosot N® is unclear.

Lymphomyosot® or Lymphomyosot N® is a complex homeopathic agent that was found in a human study to be effective in, inter alia, treating edemas of thrombotic or inflammatory aetiology (Küstermann 1997, Biologische Medizin 26(3): 110-114; Latini 1999, Biomedical Therapy 17(3): 79-82; Moyseyenko 2009, Europ J Integr Med 1(4): 251; Schmolz 2001, Bio Med 30(4): 177-183; Van Haselen 2008, Europ J Integr Med 1(Suppl. 1): S46-47; Zenner 1990, Biological Therapy 8(3): 49-53). Zenner and Metelmann describe the therapeutic use of Lymphomyosot and the results of a multicenter use observation study on 3,512 patients (Journal of Natural Medicine, (199006), vol. 3, no. 3, pages 1 - 19). However, the mechanism of action and the effectiveness for said agent is unclear.

However, there is still a need for further compositions for efficiently treating and/or preventing lymphedema.

The technical problem underlying the present invention can be seen as the provision of means and methods for complying with the aforementioned needs. The technical problem is solved by the embodiments characterized in the claims and herein below.

Accordingly, the present invention relates to a composition for use in treating and/or preventing lymphedema,
wherein said composition comprises:
i) Aranea diadematus D6 dilution;
ii) Calcium phosphoricum D12 dilution;
iii) Equisetum hiemale D4 ex herba re dilution;
iv) Ferrum jodatum D12 dilution;
v) Fumaria officinalis D4 dilution;
vi) Gentiana lutea D5 dilution;
vii) Geranium robertianum D4 dilution;
viii) Levothyroxinum D12 dilution;
ix) Myosotis arvensis D3 dilution;
x) Nasturtium officinale D4 dilution;
xi) Natrium sulfuricum D4 dilution;
xii) Pinus silvestris D4 dilution;
xiii) Sarsaparillae radix D6 dilution;
xiv) Scrophularia nodosa D3 dilution;
xv) Teucrium scorodonia D3 dilution; and
xvi) Veronica officinalis D3 dilution,
and
wherein said composition is administered in an amount of 0.25 ml per kg body weight of the subject to be treated every other day.

The term "composition" as used herein refers to a mixture of extracts from, inter alia, biological sources such as plants which are further defined elsewhere herein. Preferably, the said composition can comprise further other ingredients and, more preferably, comprises a pharmaceutically acceptable carrier and/or diluent.

Preferably, such further ingredients can be stabilizing agents, wetting agents, pharmaceutical carriers, additional pharmaceutically active agents, release controlling agents and the like. Preferred diluents encompass water, alcohols, physiological saline solutions, buffers, such as phosphate buffered saline solutions, syrup, oil, water, emulsions, various types of wetting agents, and the like. Preferably envisaged in accordance with the present invention is a composition which further comprises at least one pharmaceutically acceptable carrier and/or diluent. The carrier(s) must be acceptable in the sense of being compatible with the other ingredients of the formulation and being not deleterious to the recipient thereof. The pharmaceutical carrier employed may include a solid, a gel, or a liquid. Exemplary of solid carriers are lactose, terra alba, sucrose, talc, gelatin, agar, pectin, acacia, magnesium stearate, stearic acid and the like. Similarly, the carrier or diluent may include time delay material well known to the art, such as glyceryl mono-stearate or glyceryl distearate alone or with a wax. Said suitable carriers comprise those mentioned above and others well known in the art, see, e.g., Remington's Pharmaceutical Sciences, Mack Publishing Company, Easton, Pennsylvania, European Pharmacopeia, Homeopathic Pharmacopeia of the USA or HAB. The pharmaceutically acceptable diluent is selected so as not to affect the biological activity of the combination. Examples of such diluents are distilled water, physiological saline, Ringer's solutions, dextrose solution, and Hank's solution. In addition, the pharmaceutical composition or formulation may also include other carriers, adjuvants, or non-toxic, non-therapeutic, non-immunogenic stabilizers and the like.

The composition shall be adapted for use in treating and/or preventing lymphedema. Accordingly, it will be understood that dependent on the desired mode of administration the composition shall be formulated for a systemic or topical application. Preferably, the composition envisaged herein is formulated for a systemic or local application. Preferably, oral application, e.g. in the form of tablets, solution or drinking ampules, is envisaged or application via injection. However, depending on the nature and the mode of action, the composition may be administered by other routes as well including dermal, intra-muscular, subcutaneous, oral, intravenous or topical administration. The composition can be, preferably, formulated for a bolus administration or can be made for continuous applications as set forth elsewhere herein in detail. Preferably, the composition for use according to the present invention is formulated as a medicament as set forth elsewhere herein in detail.

The term "treating" as used herein refers to any improvement of lymphedema that occurs in a treated subject compared to an untreated subject. Such an improvement can be a prevention of a worsening or progression of the lymphedema. Preferably, treating, however, refers to curing and/or amelioration of lymphedema. It will be understood that a treatment may not be successful for 100% of the subjects to be treated. The term, however, requires that the treatment is successful for a statistically significant portion of the subjects (e.g. a cohort in a cohort study). Whether a portion is statistically significant can be determined without further ado by the person skilled in the art using various well known statistic evaluation tools, e.g., determination of confidence intervals, p-value determination, Student's t-test, Mann-Whitney test etc.. Details are found in Dowdy and Wearden, Statistics for Research, John Wiley & Sons, New York 1983. Preferred confidence intervals are at least 90%, at least 95%, at least 97%, at least 98% or at least 99 %. The p-values are, preferably, 0.05, 0.01, 0.005, or 0.0001.

The term "preventing" as used herein refers to a prevention of the formation of lymphedema within a certain time window ("preventive window"). The preventive window can be at least or up to 1 week, at least or up to 2 weeks, at least or up to 3 weeks, at least or up to 4 weeks, at least or up to 1 month, at least or up to 2 months, at least or up to 3 months, at least or up to 6 months or at least or up to 1 year after administration of the composition. It will be understood that such a prevention may not be successful for 100% of the subjects to which the composition has been administered. The term, however, requires that the prevention is successful for a statistically significant portion of the subjects (e.g. a cohort in a cohort study). Whether a portion is statistically significant can be determined without further ado by the person skilled in the art using various well known statistic evaluation tools referred to elsewhere in detail.

The term "lymphedema" as used herein refers to a disorder characterized by an increased fluid retention in the tissue, tissue swelling and an impairment of immune function due to reduced lymphatic drainage. Preferably, lymphedema is often found as secondary disorder which may result from lymph node dissection or injury of lymph vessels. Such secondary lymphedema may also accompany, e.g., lymph node dissection or injury in connection with surgery, radiation therapy, tumor disease and treatments thereof, musculoskeletal injuries like fractures, tendon releases and joint replacements, neurological conditions like muscle paresis, vascular injuries/surgeries, integumentary injuries, coagulation disorders such as deep vein thrombosis, scar tissue formation, tamoxifen treatment, filariasis, infection, lipedema or cellulitis.

Accordingly, the composition for use according to the present invention in treating and/or preventing lymphedema, preferably, reduces swelling in lymphedema by supporting the tissue regeneration process and therefore the functional lymphatic regeneration and/or by accelerating the resolution of the inflammatory process. Moreover, lymphedema in accordance with the present invention is preferably secondary lymphedema that occurs, e.g., after lymph node dissection or lymphatic vessel injury and is, most preferably, associated with cancer. The symptoms and characteristics of lymphedema are also well known to those skilled in the art and are described in more detail in standard text books of medicine such as Stedman or Pschyrembl.

The plants referred to in accordance with the present invention, i.e. Equisetum hiemale, Fumaria officinalis, Gentiana lutea, Geranium robertianum, Myosotis arvensis, Nasturtium officinale, Pinus silvestris, Sarsaparillae radix, Scrophularia nodosa, Teucrium scorodonia, Veronica officinalis, are well known to the person skilled in the art and botanically characterized. Accordingly, the said plants as a starting material for preparing the composition for use according to the present invention can be identified, cultured and/or harvested without further ado. For example, the plants can be obtained by growing developing seedlings in the green house under standard conditions of humidity, temperature, and illumination into plants, culturing said plants for a period of time sufficient to allow for production of secondary plant metabolites and harvesting the plants or parts thereof required for the extraction process defined elsewhere herein. Furthermore, the said plants as a starting material for preparing the composition for use according to the present invention can, preferably, also be obtained from wild harvesting. In this case, the plants are identified using micro- and macroscopic testing methods.

Other components of the composition are also well known chemicals in homeopathic applications, such as Calcium phosphoricum Ferrum jodatum, Levothyroxinum, Natrium sulfuricum, or animal derived extracts, such as those from Aranea diadematus (cross spider, entire animal). Again, these components, in particular as homeopathic ingredients, are well known to the person skilled in the art.

The composition for use according to the present invention comprises the said components in the following homeopathic dilutions:
i) Aranea diadematus D6 dilution;
ii) Calcium phosphoricum D12 dilution;
iii) Equisetum hiemale D4 ex herba re dilution;
iv) Ferrum jodatum D12 dilution;
v) Fumaria officinalis D4 dilution;
vi) Gentiana lutea D5 dilution;
vii) Geranium robertianum D4 dilution;
viii) Levothyroxinum D12 dilution;
ix) Myosotis arvensis D3 dilution;
x) Nasturtium officinale D4 dilution;
xi) Natrium sulfuricum D4 dilution;
xii) Pinus silvestris D4 dilution;
xiii) Sarsaparillae radix D6 dilution;
xiv) Scrophularia nodosa D3 dilution;
xv) Teucrium scorodonia D3 dilution; and
xvi) Veronica officinalis D3 dilution.

More preferably, said formulations can be obtained as follows.

The term "Aranea diadematus D6 dilution" as used herein refers to a dilution of Aranea diadematus which consists of living cross spiders, i.e. Araneus diadematus CLERCK. The basic colouring of said creatures is mostly light brown, but this colour can vary widely depending on the environment. The cross spider gets its name from the white markings in the form of a cross on the light or dark brown background of the plump which is the glossy anterior half of the abdomen. A mother tincture from this material is obtainable according to European Pharmacopoeia method 1.1.9 resp. 4b of the German Homoeopathic Pharmacopoeia using ethanol as vehicle. 1 part of living animals is anaesthetised in a suitable flask using carbon dioxide. The animals are transferred to a porcelain dish and killed with a part of ethanol (90% V/V). The creatures are crushed with a mortar. Then, sufficient ethanol (90% V/V) is added to bring the total quantity added up to 10 parts by weight. Greater amounts can be crushed using a mixer. The preparation is transferred to a lockable container and left at a temperature not exceeding 20°C for a minimum of 14 days while being repeatedly agitated. Finally, the mixture is passed through a filter. In accordance with European Pharmacopoeia method 1.1.9 resp. 4b of the German Homoeopathic Pharmacopoeia, the 2nd and 3rd decimal dilutions (D2 and D3) are carried out using ethanol (90% V/V) and the 4th decimal dilution (D4) using ethanol (70% V/V). Starting from the production of the dilution D2, always 1 part of the 1 step higher concentrated dilution and 9 parts of ethanol are used. Preferably, the Aranea diadematus mother tincture to be used for the composition for use according to the present invention is characterized by the analytical parameters set forth in the respective monograph. The dilution is mixed with the other dilutions. After twice potentisation of the mixture of the dilutions, the aforementioned mother tincture is comprised in the composition for use according to the invention, preferably, in the D6 dilution and in a portion of 0.05 % (m/m) (0.55 mg dilution D6 in one ampoule of 1.1 g).

The term "Calcium phosphoricum D12 dilution" as used herein refers to a dilution of Calcium phosphoricum which is obtainable according to the corresponding monograph of the European Pharmacopoeia. Calcium phosphoricum is a white to almost white crystalline powder. It is practically insoluble in water and in ethanol (96 %). It dissolves in dilute hydrochloric acid and in dilute nitric acid. Calcium hydrogenphosphate (CaHPO₄) is produced in a neutralization reaction between phosphoric acid (H₃PO₄) and calcium hydroxide (Ca(OH)₂). A suitable amount of calcium hydroxide (Ca(OH)₂) is added to thermal phosphoric acid (H₃PO₄). The mixture is cooled to precipitate calcium hydrogenphosphate dihydrate (CaHPO₄ • 2H₂O). The precipitate is filtered off and dried softly. The solid dilution D1 (trituration) is obtainable according to European Pharmacopoeia method 4.1.1 resp. 6 of the German Homoeopathic Pharmacopoeia using lactose monohydrate as vehicle. The starting material is comminuted and sieved before dividing in three parts. The first third of the vehicle is triturated. The starting material is added to the triturated vehicle. The mixture is triturated and scraped down. Subsequently the two equal portions of the remaining vehicle are added, triturated and scraped down. A D2 trituration is obtained by admixing 1 part of Calcium phosphoricum trituration D1 with 9 parts of lactose-monohydrate. The 3th to 6th decimal triturations are produced accordingly. One part of the trituration D6 is subsequently dissolved with 9 parts of purified water and succussed in order to obtain the dilution D7. This step is performed in compliance with Ph. Eur. method 3.2.1 resp. method 8a of the German Homoeopathic Pharmacopoeia. To obtain the dilution D8 one part of the dilution D7 is potentised with 9 parts of ethanol 30 % (m/m). The next potentisation step to obtain the dilution D9 is performed using ethanol 43 % (m/m). The 10th decimal dilution is produced accordingly. Preferably, Calcium phosphoricum to be used for the composition for use according to the present invention is characterized by the analytical parameters set forth in the respective monograph. The dilution is mixed with the other dilutions. After twice potentisation of the mixture of the dilutions, the aforementioned mother tincture is comprised in the composition for use according to the invention, preferably, in a D12 dilution and in a portion of about 0.05% (0.55 mg dilution D12 in one ampoule of 1.1g).

The term "Equisetum hiemale D4 ex herba re dilution" as used herein refers to a dilution of Equisetum hiemale to be used for the preparation of the composition for use according to the present invention. Said Equisetum hiemale dilution is an alcoholic extract obtainable according to method 1.1.3 of the European Pharmacopoeia resp. method 2a of the German Homoeopathic Pharmacopoeia. How to carry out the method is well known to the skilled artisan. Preferably, the Equisetum hiemale extract to be used in accordance with the composition for use of the present invention is prepared as follows: A mother tincture is obtained from the fresh whole plant Equisetum hiemale L. containing less than 70% of expressed juice and more than 60% moisture (loss on drying) and no essential oil or resin. Suitable herbal materials are subsequently comminuted, admixed with an amount of ethanol 90 % (V/V) for not less than half the mass of the herbal material. The precise amount of ethanol to be added is to be calculated according to the following formula: mass of herbal material (kg) multiplied by the percentage of loss on drying (this can be determined by analyzing the loss on drying for a sample of the herbal material) divided by 100. The mixture is subsequently stored in closed containers at room temperature (not exceeding 20°C) for at least ten days with swirling from time to time. The mixture is than expressed and the resulting liquid is filtered. The filtrate is adjusted with ethanol (50% V/V). The amount of ethanol for said adjustment is calculated as follows: Mass of filtrate (kg) multiplied by [percentage dry residue or percentage assay content required according to the monograph] divided by [percentage of dry residue or assay content required according to the monograph]. The adjusted mixture is allowed to stand at room temperature (not exceeding 20°C) for not less than 5 days and, optionally, filtered again to yield the mother tincture. D-Dilutions of the mother tincture can be made by applying the following dilution scheme: 2 parts of the mother tincture are admixed with 8 parts of ethanol (50% V/V) to obtain a D1 dilution. 1 part of the D1 dilution is admixed with 9 parts of ethanol (50% V/V) to obtain a D2 dilution. Subsequent decimal dilutions are produced as stated for the D2. Preferably, the Equisetum hiemale mother tincture to be used for the composition for use according to the invention is characterized by the analytical parameters set forth in the respective monograph. The dilution is mixed with the other dilutions. After twice potentisation of the mixture of the dilutions, the aforementioned mother tincture is comprised in the composition for use according to the invention, preferably, in a D4 dilution and in a portion of 0.05 % (m/m) (0.55 mg dilution D4 in one ampoule of 1.1 g).

The term "Ferrum jodatum D12 dilution" as used herein refers to a dilution of Ferrum iodatum which is a reaction product from iron and iodine dried on lactose containing no less than 15.0 and no more than 25.0 per cent FeI₂ when calculated with reference to the anhydrous substance. Pour 10 parts of water over 3 parts of Ferrum metallicum and gradually add 8 parts of iodine. Allow the mixture to stand for 15 hours over night, boil until the solution turns a clear green colour and filter the solution obtained onto 40 parts lactose. Dry in a vacuum for 24 hours at room temperature. Mix the mass thoroughly several times while it is drying. Then triturate the mixture. The solid dilution D2 (trituration) is obtainable according to Ph. Eur. method 4.1.1 resp. 6 of the German Homoeopathic Pharmacopoeia using lactose monohydrate as vehicle. The material is potentised twice in order to obtain a dilution D2. Further liquid dilutions are obtained according to Ph. Eur. method 3.2.1 resp. method 8a of the German Homoeopathic Pharmacopoeia using purified water or ethanol of different concentrations. The Dilutions can be made by applying the following dilution scheme: A D3 trituration is obtained by admixing 1 part of Ferrum iodatum trituration D2 with 9 parts of lactose-monohydrate. The 4th to 6th decimal triturations are produced accordingly. One part of the trituration D6 is subsequently dissolved with 9 parts of purified water and succussed in order to obtain the dilution D7. This step is performed in compliance with Ph. Eur. method 3.2.1 resp. method 8a of the German Homoeopathic Pharmacopoeia. To obtain the dilution D8 one part of the dilution D7 is potentised with 9 parts of ethanol 30 % (m/m). The next potentisation step to obtain the dilution D9 is performed using ethanol 43 % (m/m). The 10th decimal dilution is produced accordingly. Preferably, Ferrum iodatum to be used for the composition for use according to the present invention is characterized by the analytical parameters set forth in the respective monograph. After mixing of the dilution with the other dilutions and twice potentisation, Ferrum iodatum is comprised in the composition for use according to the invention, preferably, in a D12 dilution and in a portion of about 0.1 % (1.1 mg dilution D12 in one ampoule of 1.1g).

The term "Fumaria officinalis D4 dilution" as used herein refers to a dilution of Fumaria officinalis to be used for the preparation of the composition for use according to the present invention. Said Fumaria officinalis dilution is an alcoholic extract obtainable according to method 1.1.3 of the European Pharmacopoeia resp. method 2a of the German Homoeopathic Pharmacopoeia. How to carry out the method is well known to the skilled artisan. Preferably, the Fumaria officinalis extract to be used in accordance with the composition for use of the present invention is prepared as follows: A mother tincture is obtained from the fresh aerial parts of Fumaria officinalis L. at flowering time containing less than 70% of expressed juice and more than 60% moisture (loss on drying) and no essential oil or resin. Suitable herbal materials are subsequently comminuted, admixed with an amount of ethanol 90 % (V/V) for not less than half the mass of the herbal material. The precise amount of ethanol to be added is to be calculated according to the following formula: mass of herbal material (kg) multiplied by the percentage of loss on drying (this can be determined by analyzing the loss on drying for a sample of the herbal material) divided by 100. The mixture is subsequently stored in closed containers at room temperature (not exceeding 20°C) for at least ten days with swirling from time to time. The mixture is than expressed and the resulting liquid is filtered. The filtrate is adjusted with ethanol (50% V/V). The amount of ethanol for said adjustment is calculated as follows: Mass of filtrate (kg) multiplied by [percentage dry residue or percentage assay content required according to the monograph] divided by [percentage of dry residue or assay content required according to the monograph]. The adjusted mixture is allowed to stand at room temperature (not exceeding 20°C) for not less than 5 days and, optionally, filtered again to yield the mother tincture. D-Dilutions of the mother tincture can be made by applying the following dilution scheme: 2 parts of the mother tincture are admixed with 8 parts of ethanol (50% V/V) to obtain a D1 dilution. 1 part of the D1 dilution is admixed with 9 parts of ethanol (50% V/V) to obtain a D2 dilution. Subsequent decimal dilutions are produced as stated for the D2. Preferably, the Fumaria officinalis mother tincture to be used for the composition for use according to the invention is characterized by the analytical parameters set forth in the respective monograph. The dilution is mixed with the other dilutions. After twice potentisation of the mixture of the dilutions, the aforementioned mother tincture is comprised in the composition for use according to the invention, preferably, in a D4 dilution and in a portion of 0.05 % (m/m) (0.55 mg dilution D4 in one ampoule of 1.1 g).

The term "Gentiana lutea D5 dilution" as used herein refers to a dilution of Gentiana lutea to be used for the preparation of the composition for use according to the present invention. Said Gentiana lutea dilution is an alcoholic extract obtainable according to method 1.1.5 of the European Pharmacopoeia resp. method 3a of the German Homoeopathic Pharmacopoeia. How to carry out the method is well known to the skilled artisan. Preferably, the Gentiana lutea extract to be used in accordance with the composition for use of the present invention is prepared as follows: A mother tincture is obtained from the fresh underground parts of Gentiana lutea L. containing essential oil re resin, or generally less than 60% moisture (loss on drying). Suitable herbal materials are subsequently comminuted, admixed with an amount of ethanol 90 % (V/V) for not less than half the mass of the herbal material. The precise amount of ethanol to be added is to be calculated according to the following formula: mass of herbal material (kg) multiplied by the percentage of loss on drying (this can be determined by analyzing the loss on drying for a sample of the herbal material), multiplied by a factor 2 and divided by 100. The mixture is subsequently stored in closed containers at room temperature (not exceeding 20°C) for at least ten days with swirling from time to time. The mixture is than expressed and the resulting liquid is filtered. The filtrate is adjusted with ethanol (70% V/V). The amount of ethanol for said adjustment is calculated as follows: Mass of filtrate (kg) multiplied by [percentage dry residue or percentage assay content required according to the monograph] divided by [percentage of dry residue or assay content required according to the monograph]. The adjusted mixture is allowed to stand at room temperature (not exceeding 20°C) for not less than 5 days and, optionally, filtered again to yield the mother tincture. D-Dilutions of the mother tincture can be made by applying the following dilution scheme: 3 parts of the mother tincture are admixed with 7 parts of ethanol (70% V/V) to obtain a D1 dilution. 1 part of the D1 dilution is admixed with 9 parts of ethanol (70% V/V) to obtain a D2 dilution. Subsequent decimal dilutions are produced as stated for the D2. Preferably, the Gentiana lutea mother tincture to be used for the composition for use according to the invention is characterized by the analytical parameters set forth in the respective monograph. The dilution is mixed with the other dilutions. After twice potentisation of the mixture of the dilutions, the aforementioned mother tincture is comprised in the composition for use according to the invention, preferably, in a D5 dilution and in a portion of 0.05 % (m/m) (0.55 mg dilution D5 in one ampoule of 1.1 g).

The term "Geranium robertianum D4 dilution" as used herein refers to a dilution of Geranium robertianum to be used for the preparation of the composition for use according to the present invention. Said Geranium robertianum dilution is an alcoholic extract obtainable according to method 1.1.3 of the European Pharmacopoeia resp. method 2a of the German Homoeopathic Pharmacopoeia. How to carry out the method is well known to the skilled artisan. Preferably, the Geranium robertianum extract to be used in accordance with the composition for use of the present invention is prepared as follows: A mother tincture is obtained from the fresh aerial parts of Equisetum hiemale L. at flowering time containing less than 70% of expressed juice and more than 60% moisture (loss on drying) and no essential oil or resin. Suitable herbal materials are subsequently comminuted, admixed with an amount of ethanol 90 % (V/V) for not less than half the mass of the herbal material. The precise amount of ethanol to be added is to be calculated according to the following formula: mass of herbal material (kg) multiplied by the percentage of loss on drying (this can be determined by analyzing the loss on drying for a sample of the herbal material) divided by 100. The mixture is subsequently stored in closed containers at room temperature (not exceeding 20°C) for at least ten days with swirling from time to time. The mixture is than expressed and the resulting liquid is filtered. The filtrate is adjusted with ethanol (50% V/V). The amount of ethanol for said adjustment is calculated as follows: Mass of filtrate (kg) multiplied by [percentage dry residue or percentage assay content required according to the monograph] divided by [percentage of dry residue or assay content required according to the monograph]. The adjusted mixture is allowed to stand at room temperature (not exceeding 20°C) for not less than 5 days and, optionally, filtered again to yield the mother tincture. D-Dilutions of the mother tincture can be made by applying the following dilution scheme: 2 parts of the mother tincture are admixed with 8 parts of ethanol (50% V/V) to obtain a D1 dilution. 1 part of the D1 dilution is admixed with 9 parts of ethanol (50% V/V) to obtain a D2 dilution. Subsequent decimal dilutions are produced as stated for the D2. Preferably, the Geranium robertianum mother tincture to be used for the composition for use according to the invention is characterized by the analytical parameters set forth in the respective monograph. The dilution is mixed with the other dilutions. After twice potentisation of the mixture of the dilutions, the aforementioned mother tincture is comprised in the composition for use according to the invention, preferably, in a D4 dilution and in a portion of 1.1 % (m/m) (1.10 mg dilution D4 in one ampoule of 1.1 g).

The term "Levothyroxinum D12 dilution" as used herein refers to a dilution of Levothyroxinum which contains not less than 97.0 and not more than 102.0 percent of (S)-2-amino-3-[4-(4-hydroxy-3,5-diiodophenoxy)-3,5-diiodophenyl]-propionic acid when calculated with reference to the anhydrous substance. It is a reaction product from 3,5-diiodo-L-thyronine which is iodinated to the intermediate 3,3'5,5'-tetraiodo-L-thyronine. The free acid is obtained by wetting with ethanol. After a drying process and mixing the final product is available. The solid dilution D1 (trituration) is obtainable according to European Pharmacopoeia method 4.1.1 resp. method 6 of the German Homoeopathic Pharmacopoeia and the corresponding company own monograph using lactose monohydrate as vehicle. The starting material is comminuted and sieved before dividing in three parts. The first third of the vehicle is triturated. The starting material is added to the triturated vehicle. The mixture is triturated and scraped down. Subsequently the two equal portions of the remaining vehicle are added, triturated and scraped down. A D2 trituration is obtained by admixing 1 part of Levothyroxinum trituration D1 with 9 parts of lactose-monohydrate. The 3th to 6th decimal triturations are produced accordingly. One part of the trituration D6 is subsequently dissolved with 9 parts of purified water and succussed in order to obtain the dilution D7. This step is performed in compliance with Ph. Eur. method 3.2.1 resp. method 8a of the German Homoeopathic Pharmacopoeia. To obtain the dilution D8 one part of the dilution D7 is potentised with 9 parts of ethanol 30 % (m/m). The next potentisation step to obtain the dilution D9 is performed using ethanol 43 % (m/m). The 10th decimal dilution is produced accordingly. Preferably, Levothyroxinum to be used for the composition for use according to the present invention is characterized by the analytical parameters set forth in the respective monograph. The dilution is mixed with the other dilutions. After twice potentisation of the mixture of the dilutions, Levothyroxinum is comprised in the composition for use according to the invention, preferably, in a D12 dilution and in a portion of about 0.05% (0.55 mg dilution D12 in one ampoule of 1.1g).

The term "Myosotis arvensis D3 dilution" as used herein refers to a dilution of Myosotis arvensis to be used for the preparation of the composition for use according to the present invention. Said Myosotis arvensis dilution is an alcoholic extract obtainable according to method 1.1.5 of the European Pharmacopoeia resp. method 3a of the German Homoeopathic Pharmacopoeia. How to carry out the method is well known to the skilled artisan. Preferably, the Myosotis arvensis extract to be used in accordance with the composition for use of the present invention is prepared as follows: A mother tincture is obtained from the fresh aerial parts of Myosotis arvensis a L. at flowering containing essential oil re resin, or generally less than 60% moisture (loss on drying). Suitable herbal materials are subsequently comminuted, admixed with an amount of ethanol 90 % (V/V) for not less than half the mass of the herbal material. The precise amount of ethanol to be added is to be calculated according to the following formula: mass of herbal material (kg) multiplied by the percentage of loss on drying (this can be determined by analyzing the loss on drying for a sample of the herbal material), multiplied by a factor 2 and divided by 100. The mixture is subsequently stored in closed containers at room temperature (not exceeding 20°C) for at least ten days with swirling from time to time. The mixture is than expressed and the resulting liquid is filtered. The filtrate is adjusted with ethanol (70% V/V). The amount of ethanol for said adjustment is calculated as follows: Mass of filtrate (kg) multiplied by [percentage dry residue or percentage assay content required according to the monograph] divided by [percentage of dry residue or assay content required according to the monograph]. The adjusted mixture is allowed to stand at room temperature (not exceeding 20°C) for not less than 5 days and, optionally, filtered again to yield the mother tincture. D-Dilutions of the mother tincture can be made by applying the following dilution scheme: 3 parts of the mother tincture are admixed with 7 parts of ethanol (70% V/V) to obtain a D1 dilution. Preferably, the Myosotis arvensis mother tincture to be used for the composition for use according to the invention is characterized by the analytical parameters set forth in the respective monograph. The dilution is mixed with the other dilutions. After twice potentisation of the mixture of the dilutions, the aforementioned mother tincture is comprised in the composition for use according to the invention, preferably, in a D3 dilution and in a portion of 0.05 % (m/m) (0.55 mg dilution D3 in one ampoule of 1.1 g).

The term "Nasturtium officinale D4 dilution" as used herein refers to a dilution of Nasturtium officinale to be used for the preparation of the composition for use according to the present invention. Said Nasturtium officinale dilution is an alcoholic extract obtainable according to method 1.1.5 of the European Pharmacopoeia resp. method 3a of the German Homoeopathic Pharmacopoeia. How to carry out the method is well known in the art. Preferably, the Nasturtium officinale extract to be used in accordance with the composition for use of the present invention is prepared as follows: A mother tincture is obtained from the fresh aerial parts of Nasturtium officinale R. Br. at flowering containing essential oil re resin, or generally less than 60% moisture (loss on drying). Suitable herbal materials are subsequently comminuted, admixed with an amount of ethanol 90 % (V/V) for not less than half the mass of the herbal material. The precise amount of ethanol to be added is to be calculated according to the following formula: mass of herbal material (kg) multiplied by the percentage of loss on drying (this can be determined by analyzing the loss on drying for a sample of the herbal material), multiplied by a factor 2 and divided by 100. The mixture is subsequently stored in closed containers at room temperature (not exceeding 20°C) for at least ten days with swirling from time to time. The mixture is then expressed and the resulting liquid is filtered. The filtrate is adjusted with ethanol (70% V/V). The amount of ethanol for said adjustment is calculated as follows: Mass of filtrate (kg) multiplied by [percentage dry residue or percentage assay content required according to the monograph] divided by [percentage of dry residue or assay content required according to the monograph]. The adjusted mixture is allowed to stand at room temperature (not exceeding 20°C) for not less than 5 days and, optionally, filtered again to yield the mother tincture. D-Dilutions of the mother tincture can be made by applying the following dilution scheme: 3 parts of the mother tincture are admixed with 7 parts of ethanol (70% V/V) to obtain a D1 dilution. 1 part of the D1 dilution is admixed with 9 parts of ethanol (70% V/V) to obtain a D2 dilution. Subsequent decimal dilutions are produced as stated for the D2. Preferably, the Nasturtium officinale mother tincture to be used for the composition for use according to the invention is characterized by the analytical parameters set forth in the respective monograph. The dilution is mixed with the other dilutions. After twice potentisation of the mixture of the dilutions, the aforementioned mother tincture is comprised in the composition for use according to the invention, preferably, in a D4 dilution and in a portion of 1.1 % (m/m) (1.10 mg dilution D4 in one ampoule of 1.1 g).

The term "Natrium sulfuricum D4 dilution" as used herein refers to a dilution of Natrium sulfuricum obtainable according to the corresponding European Pharamcopoeia monograph is a reaction product from Sodium chloride and sulphuric acid containing 98.5 per cent to 101.0 per cent Natrium sulfuricum. Sodium sulphate (Na2SO4) is produced from sodium chloride (NaCl) and sulphuric acid (H₂SO₄) in a water cooled furnace. Sodium sulphate hydrate (Na₂SO₄•xH₂O) precipitates. The product is filtered off and purified by recrystallisation from water. The sodium sulphate hydrate (Na₂SO₄•xH₂O) is then thermally dried. The solution D1 (liquid) is obtainable according to Ph. Eur. method 3.1.1 resp. 5a of the German Homoeopathic Pharmacopoeia using ethanol as vehicle. Since the material has a insufficient solubility, the dilution D2 is produced directly. 1 part of the starting material is dissolved in 99 parts of ethanol (18% V/V). Preferably, Natrium sulfuricum to be used for the composition for use according to the present invention is characterized by the analytical parameters set forth in the respective monograph. After mixing of the dilution with the other dilutions and twice potentisation, Natrium sulfuricum is comprised in the composition for use according to the invention, preferably, in a D4 dilution and in a portion of about 0.05% (0.55 mg dilution D4 in one ampoule of 1.1g).

The term "Pinus silvestris D4 dilution" as used herein refers to a dilution of Pinus silvestris to be used for the preparation of the composition for use according to the present invention. Said Pinus silvestris dilution is an alcoholic extract obtainable according to method 1.1.5 of the European Pharmacopoeia resp. method 3a of the German Homoeopathic Pharmacopoeia. How to carry out the method is well known to the skilled artisan. Preferably, the Pinus silvestris extract to be used in accordance with the composition for use of the present invention is prepared as follows: A mother tincture is obtained from the fresh shoots of Pinus silvestris L. up to 50 mm long, collected during growing season. The material contains essential oil re resin, or generally less than 60% moisture (loss on drying). Suitable herbal materials are subsequently comminuted, admixed with an amount of ethanol 90 % (V/V) for not less than half the mass of the herbal material. The precise amount of ethanol to be added is to be calculated according to the following formula: mass of herbal material (kg) multiplied by the percentage of loss on drying (this can be determined by analyzing the loss on drying for a sample of the herbal material), multiplied by a factor 2 and divided by 100. The mixture is subsequently stored in closed containers at room temperature (not exceeding 20°C) for at least ten days with swirling from time to time. The mixture is than expressed and the resulting liquid is filtered. The filtrate is adjusted with ethanol (70% V/V). The amount of ethanol for said adjustment is calculated as follows: Mass of filtrate (kg) multiplied by [percentage dry residue or percentage assay content required according to the monograph] divided by [percentage of dry residue or assay content required according to the monograph]. The adjusted mixture is allowed to stand at room temperature (not exceeding 20°C) for not less than 5 days and, optionally, filtered again to yield the mother tincture. D-Dilutions of the mother tincture can be made by applying the following dilution scheme: 3 parts of the mother tincture are admixed with 7 parts of ethanol (70% V/V) to obtain a D1 dilution. 1 part of the D1 dilution is admixed with 9 parts of ethanol (70% V/V) to obtain a D2 dilution. Subsequent decimal dilutions are produced as stated for the D2. Preferably, the Pinus silvestris mother tincture to be used for the composition for use according to the invention is characterized by the analytical parameters set forth in the respective monograph. The dilution is mixed with the other dilutions. After twice potentisation of the mixture of the dilutions, the aforementioned mother tincture is comprised in the composition for use according to the invention, preferably, in a D4 dilution and in a portion of 0.05 % (m/m) (0.55 mg dilution D4 in one ampoule of 1.1 g).

The term "Sarsaparillae radix D6 dilution" as used herein refers to a dilution of Sarsaparillae radix (Smilax) to be used for the preparation of the composition for use according to the present invention. Said Sarsaparillae radix dilution is an alcoholic extract obtainable according to method 1.1.8 of the European Pharmacopoeia resp. method 4a of the German Homoeopathic Pharmacopoeia. How to carry out the method is well known to the skilled artisan. The method is generally used for dried herbal drugs. Preferably, the Smilax extract to be used in accordance with the composition for use of the present invention is prepared as follows: A mother tincture is obtained from the dried underground parts of Smilax regelii Kill. Et C.V. Morton and Smilax medica Schlechtend. et Cham. or other related species. The macerate is prepared by mixing 1 part of the comminuted dried plant and 10 parts of ethanol of the appropriate concentration (70% V/V). The mixture is allowed to stand in a closed container for not less than 10 days at a temperature not exceeding 25 °C. After the maceration, the extraction solvent is separated from the residue by decantation. If necessary the residue is pressed out. The precise amount of ethanol to be added is to be calculated according to the following formula: mass of macerate (kg) multiplied by the difference of [percentage dry residue or percentage assay content as required in the individual monograph] - [percentage dry residue or percentage assay content of the filtrate]. The calculated amount of ethanol is mixed with the filtrate. The adjusted mixture is allowed to stand at room temperature (not exceeding 20°C) for not less than 5 days and, optionally, filtered again to yield the mother tincture. The mother tincture corresponds to the 1 st decimal dilution (D 1). D-Dilutions of the mother tincture can be made by applying the following dilution scheme: 1 parts of the mother tincture are admixed with 9 parts of ethanol (70% V/V) to obtain a D2 dilution. The 3rd decimal dilution is produced accordingly. The 4th decimal dilution is produced using ethanol (50% V/V) as vehicle. Preferably, the Smilax mother tincture to be used for the composition for use according to the invention is characterized by the analytical parameters set forth in the respective monograph. The dilution is mixed with the other dilutions. After twice potentisation of the mixture of the dilutions, the aforementioned mother tincture is comprised in the composition for use according to the invention, preferably, in a D6 dilution and in a portion of 0.05 % (m/m) (0.55 mg dilution D6 in one ampoule of 1.1 g).

The term "Scrophularia nodosa D3 dilution" as used herein refers to a dilution of Scrophularia nodosa to be used for the preparation of the composition for use according to the present invention. Said Scrophularia nodosa dilution is an alcoholic extract obtainable according to method 1.1.5 of the European Pharmacopoeia resp. method 3a of the German Homoeopathic Pharmacopoeia. How to carry out the method is well known to the skilled artisan. Preferably, the Scrophularia nodosa extract to be used in accordance with the composition for use of the present invention is prepared as follows: A mother tincture is obtained from the fresh aerial parts of Scrophularia nodosa L. before flowering containing essential oil re resin, or generally less than 60% moisture (loss on drying). Suitable herbal materials are subsequently comminuted, admixed with an amount of ethanol 90 % (V/V) for not less than half the mass of the herbal material. The precise amount of ethanol to be added is to be calculated according to the following formula: mass of herbal material (kg) multiplied by the percentage of loss on drying (this can be determined by analyzing the loss on drying for a sample of the herbal material), multiplied by a factor 2 and divided by 100. The mixture is subsequently stored in closed containers at room temperature (not exceeding 20°C) for at least ten days with swirling from time to time. The mixture is than expressed and the resulting liquid is filtered. The filtrate is adjusted with ethanol (70% V/V). The amount of ethanol for said adjustment is calculated as follows: Mass of filtrate (kg) multiplied by [percentage dry residue or percentage assay content required according to the monograph] divided by [percentage of dry residue or assay content required according to the monograph]. The adjusted mixture is allowed to stand at room temperature (not exceeding 20°C) for not less than 5 days and, optionally, filtered again to yield the mother tincture. D-Dilutions of the mother tincture can be made by applying the following dilution scheme: 3 parts of the mother tincture are admixed with 7 parts of ethanol (70% V/V) to obtain a D1 dilution. Preferably, the Scrophularia nodosa mother tincture to be used for the composition for use according to the invention is characterized by the analytical parameters set forth in the respective monograph. The dilution is mixed with the other dilutions. After twice potentisation of the mixture of the dilutions, the aforementioned mother tincture is comprised in the composition for use according to the invention, preferably, in a D3 dilution and in a portion of 0.05 % (m/m) (0.55 mg dilution D3 in one ampoule of 1.1 g).

The term "Teucrium scorodonia D3 dilution" as used herein refers to a dilution of Teucrium scorodonia to be used for the preparation of the composition for use according to the present invention. Said Teucrium scorodonia dilution is an alcoholic extract obtainable according to method 1.1.5 of the European Pharmacopoeia resp. method 3a of the German Homoeopathic Pharmacopoeia. How to carry out the method is well known to the skilled artisan. Preferably, the Teucrium scorodonia extract to be used in accordance with the composition for use of the present invention is prepared as follows: A mother tincture is obtained from the fresh aerial parts of flowering Teucrium scorodonia L. containing essential oil re resin, or generally less than 60% moisture (loss on drying). Suitable herbal materials are subsequently comminuted, admixed with an amount of ethanol 90 % (V/V) for not less than half the mass of the herbal material. The precise amount of ethanol to be added is to be calculated according to the following formula: mass of herbal material (kg) multiplied by the percentage of loss on drying (this can be determined by analyzing the loss on drying for a sample of the herbal material), multiplied by a factor 2 and divided by 100. The mixture is subsequently stored in closed containers at room temperature (not exceeding 20°C) for at least ten days with swirling from time to time. The mixture is then expressed and the resulting liquid is filtered. The filtrate is adjusted with ethanol (70% V/V). The amount of ethanol for said adjustment is calculated as follows: Mass of filtrate (kg) multiplied by [percentage dry residue or percentage assay content required according to the monograph] divided by [percentage of dry residue or assay content required according to the monograph]. The adjusted mixture is allowed to stand at room temperature (not exceeding 20°C) for not less than 5 days and, optionally, filtered again to yield the mother tincture. D-Dilutions of the mother tincture can be made by applying the following dilution scheme: 3 parts of the mother tincture are admixed with 7 parts of ethanol (70% V/V) to obtain a D1 dilution. Preferably, the Teucrium scorodonia mother tincture to be used for the composition for use according to the invention is characterized by the analytical parameters set forth in the respective monograph. The dilution is mixed with the other dilutions. After twice potentisation of the mixture of the dilutions, the aforementioned mother tincture is comprised in the composition for use according to the invention, preferably, in a D3 dilution and in a portion of 0.05 % (m/m) (0.55 mg dilution D3 in one ampoule of 1.1 g).

The term "Veronica officinalis D3 dilution" as used herein refers to a dilution of Veronica officinalis to be used for the preparation of the composition for use according to the present invention. Said Veronica officinalis dilution is an alcoholic extract obtainable according to method 1.1.3 of the European Pharmacopoeia resp. method 2a of the German Homoeopathic Pharmacopoeia. How to carry out the method is well known to the skilled artisan. Preferably, the Veronica officinalis extract to be used in accordance with the composition for use of the present invention is prepared as follows: A mother tincture is obtained from the fresh aerial parts of Veronica officinalis L. at flowering time containing less than 70% of expressed juice and more than 60% moisture (loss on drying) and no essential oil or resin. Suitable herbal materials are subsequently comminuted, admixed with an amount of ethanol 90 % (V/V) for not less than half the mass of the herbal material. The precise amount of ethanol to be added is to be calculated according to the following formula: mass of herbal material (kg) multiplied by the percentage of loss on drying (this can be determined by analyzing the loss on drying for a sample of the herbal material) divided by 100. The mixture is subsequently stored in closed containers at room temperature (not exceeding 20°C) for at least ten days with swirling from time to time. The mixture is then expressed and the resulting liquid is filtered. The filtrate is adjusted with ethanol (50% V/V). The amount of ethanol for said adjustment is calculated as follows: Mass of filtrate (kg) multiplied by [percentage dry residue or percentage assay content required according to the monograph] divided by [percentage of dry residue or assay content required according to the monograph]. The adjusted mixture is allowed to stand at room temperature (not exceeding 20°C) for not less than 5 days and, optionally, filtered again to yield the mother tincture. D-Dilutions of the mother tincture can be made by applying the following dilution scheme: 2 parts of the mother tincture are admixed with 8 parts of ethanol (50% V/V) to obtain a D1 dilution. Preferably, the Veronica officinalis mother tincture to be used for the composition for use according to the invention is characterized by the analytical parameters set forth in the respective monograph. The dilution is mixed with the other dilutions. After twice potentisation of the mixture of the dilutions, the aforementioned mother tincture is comprised in the composition for use according to the invention, preferably, in a D3 dilution and in a portion of 0.05 % (m/m) (0.55 mg dilution D3 in one ampoule of 1.1 g).

Preferably, the composition to be applied according to the present invention is prepared by admixing the aforementioned ingredients as follows: For an ampoule of 1.1g, Aranea diadematus D6 dilution (0.55 mg), Calcium phosphoricum D12 dilution (0.55 mg), Equisetum hiemale D4 ex herba re dilution (0.55 mg), Ferrum jodatum D12 dilution (1.10 mg), Fumaria officinalis D4 dilution (0.55 mg), Gentiana lutea D5 dilution (0.55 mg), Geranium robertianum D4 dilution (1.10 mg), Levothyroxinum D12 dilution (0.55 mg), Myosotis arvensis D3 dilution (0.55 mg), Nasturtium officinale D4 dilution (1.10 mg), Natrium sulfuricum D4 dilution (0.55 mg), Pinus silvestris D4 dilution (0.55 mg), Sarsaparillae radix D6 dilution (0.55 mg), Scrophularia nodosa D3 dilution (0.55 mg), Teucrium scorodonia D3 dilution (0.55 mg) and Veronica officinalis D3 dilution (0.55 mg) are mixed together according to Method 16 of the Organon of Homeopathy ("Homöopthisches Arzneibuch" HAB), edition 2011. The mixture obtained is potentiated twice with water for injections according to Method 11 HAB in combination with Method 5.1.1, Ph. Eur./40a, HAB. In a next step, the potentiated mixture is mixed with water for injections according to Method 16, HAB. In order to adjust the Osmolarity of the product, Sodium chloride is added and dissolved by mixing. After release, the bulk is filtrated into a sterile bag for single use utilizing a thyrus filter with an average size of the pores of the membranes of 0.2 µm and filled by a filling machine into glass ampoules. The ampoules are sterilized in large room sterilizers according to the Ph. Eur. Labelling of the ampoules with self sticking labels (heat application) utilizing labelling machines.

More preferably, a composition which can be used for treating and/or preventing lymphedema according to the present invention is the composition Lymphomyosot N®. Said Lymphomyosot N® is, however, to be applied in a dosage which is at least 18 times higher than the recommended dosage for the homeopathic applications.

The precise individually recommended dosage, in principle, may depend on further parameters well known to those skilled in the art. For example, children may receive a different dosage compared to adults. Whether the dosage needs to be adapted can be determined without further ado by the person skilled in the art using various well known calculation tools.

The composition shall be formulated and/or used or administered in a manner as to provide the components in the aforementioned dilutions to the subject in a dosage corresponding to a dosage of 0.25 ml/kg body weight of the subject every other day. The lowest experimental dosage used in mice was 17.5 times higher than the homeopathic standard use in humans which has been defined elsewhere herein. Thus, assuming a body weight of 20 g per mouse, Lymphomyosot N® was applied with 0.25 ml/kg every other day. The equivalent dosage treatment in a human being exhibiting a body weight of about 70 kg would be a minimum of 17.5 ml every other day. Preferably, the aforementioned dosage can be provided by a single administration (bolus) or may be achieved by more than one administration steps within the day. Moreover, it is also envisaged that the dosage is, preferably, provided by using continuous release devices which provide for a continuous administration of the dosage over the day. How the composition can be formulated in a proper manner in order to provide the aforementioned daily dosage is well known in the art. For example, low dosage providing compositions comprising the ingredients of the composition for use of the present invention and, preferably, the commercially available composition Lymphomyosot N®, can be administered repeatedly and, thereby, provide the required dosage. Alternatively, a composition being enriched in the individual ingredients can be provided. Such a composition would, preferably, provide the dosage upon a single bolus administration.

As discussed before, the composition for use of the present invention may also encompass further ingredients such as further pharmaceutically active ingredients. Preferably envisaged is a composition for use according to the invention as described above. Said further pharmaceutically active ingredient is Juglans regia or another Juglans species. More preferably, a Juglans regia D3 dilution is used. Such further ingredients may be comprised in the composition itself, i.e. be a component of the mixture, or may be formulated for a combined application as a physically separate entity such that the actual composition for treating lymphedema is actually formed during the combine administration process in the subject.

The term "Juglans regia D3 dilution" as used herein refers to a dilution of Juglans regia fructus cortex/folia to be used for the preparation of the composition for use according to the present invention. Said Juglans regia dilution is an alcoholic extract obtainable according to method 1.1.5 of the European Pharmacopoeia resp. method 3a of the German Homoeopathic Pharmacopoeia. How to carry out the method is well known to the skilled artisan. Preferably, the Juglans regia extract to be used in accordance with the composition for use of the present invention is prepared as follows: A mother tincture is obtained from fresh green peel of the fruit and the leaves of Juglans regia L. ssp. Regia containing essential oil re resin, or generally less than 60% moisture (loss on drying). Suitable herbal materials are subsequently comminuted, admixed with an amount of ethanol 90 % (V/V) for not less than half the mass of the herbal material. The precise amount of ethanol to be added is to be calculated according to the following formula: mass of herbal material (kg) multiplied by the percentage of loss on drying (this can be determined by analyzing the loss on drying for a sample of the herbal material), multiplied by a factor 2 and divided by 100. The mixture is subsequently stored in closed containers at room temperature (not exceeding 20°C) for at least ten days with swirling from time to time. The mixture is than expressed and the resulting liquid is filtered. The filtrate is adjusted with ethanol (70% V/V). The amount of ethanol for said adjustment is calculated as follows: Mass of filtrate (kg) multiplied by [percentage dry residue or percentage assay content required according to the monograph] divided by [percentage of dry residue or assay content required according to the monograph]. The adjusted mixture is allowed to stand at room temperature (not exceeding 20°C) for not less than 5 days and, optionally, filtered again to yield the mother tincture. D-Dilutions of the mother tincture can be made by applying the following dilution scheme: 3 parts of the mother tincture are admixed with 7 parts of ethanol (70% V/V) to obtain a D1 dilution. Preferably, the Juglans regia mother tincture to be used for the composition for use according to the invention is characterized by the analytical parameters set forth in the respective monograph. The dilution is mixed with the other dilutions. After twice potentisation of the mixture of the dilutions, the aforementioned mother tincture is comprised in the composition for use according to the invention, preferably, in a D3 dilution and in a portion of 0.05 % (m/m) (0.55 mg dilution D3 in one ampoule of 1.1 g).

Advantageously, it has been found in accordance with the present invention that a composition comprising the aforementioned ingredients is well suited for treating and/or preventing lymphedema when applied within a certain dosage range outside the homeopathic dosage used in the known medical indications. In particular, an effective dosage according to the present invention was the approx. 17.5 (5 µl) fold dosage used in homeopathic applications, i.e. a high dosage. In the studies underlying this invention, it was found that Lymphomyosot N® reduced tissue swelling in a mouse tail model of secondary lymphedema. The results suggest that Lymphomyosot N® may be effective in the treating and/or preventing lymphedema in humans.

Also described is a medicament comprising a composition,
wherein said composition comprises:
i) Aranea diadematus D6 dilution;
ii) Calcium phosphoricum D12 dilution;
iii) Equisetum hiemale D4 ex herba re dilution;
iv) Ferrum jodatum D12 dilution;
v) Fumaria officinalis D4 dilution;
vi) Gentiana lutea D5 dilution;
vii) Geranium robertianum D4 dilution;
viii) Levothyroxinum D12 dilution;
ix) Myosotis arvensis D3 dilution;
x) Nasturtium officinale D4 dilution;
xi) Natrium sulfuricum D4 dilution;
xii) Pinus silvestris D4 dilution;
xiii) Sarsaparillae radix D6 dilution;
xiv) Scrophularia nodosa D3 dilution;
xv) Teucrium scorodonia D3 dilution; and
xvi) Veronica officinalis D3 dilution,
and
wherein said composition to be administered provides the composition in an amount corresponding to the amount provided by at least about 0.125 to 0.25 ml per kg body weight of the subject to be treated every other day.

Preferably, said composition provides each ingredient either in an amount of potentiated mother tincture (column: "amount of potency") or in an amount of mother tincture (column: "amount of mother tincture") as indicated in Table 1, below.

**Table 1: Amounts of ingredients in Lymphomyosot N®**

| **Ingredient** | **Potency** | **Amount of Potency in weight** | **Amount mother tincture in weight** |
|---|---|---|---|
| Myosotis arvensis | D3 | 0,55 mg | 1,65 µg |
| Veronica officinalis | D3 | 0,55 mg | 1,1 µg |
| Teucrium scorodonia | D3 | 0,55 mg | 1,65 µg |
| Pinus sylvestris | D4 | 0,55 mg | 0,165 µg |
| Gentiana lutea | D5 | 0,55 mg | 0,0165 µg |
| Equisetum hiemale | D4 | 0,55 mg | 0,11 µg |
| Smilax | D6 | 0,55 mg | 0,0055 µg |
| Scrophularia nodosa | D3 | 0,55 mg | 1,65 µg |
| Calcium phosphoricum | D12 | 0,55 mg | 0,55 x10⁻¹² µg |
| Natrium sulfuricum | D4 | 0,55 mg | 0,055 µg |
| Fumaria officinalis | D4 | 0,55 mg | 0,11 µg |
| Levothyroxinum | D12 | 0,55 mg | 0,55 x10⁻¹² µg |
| Aranea diadema | D6 | 0,55 mg | 0,0055 µg |
| Geranium robertianum | D6 | 1,1 mg | 0,0022 µg |
| Nasturtium officinale | D4 | 1,1 mg | 0,33 µg |
| Ferrum jodatum | D12 | 1,1 mg | 1,1 x10⁻¹²µg |

| | | | |
|---|---|---|---|
| Note: The amounts for potentiated mother tincture or mother tincture are calculated for a 1.1 g ampoule. | | | |

The term "medicament" as used herein refers to a pharmaceutical composition containing the aforementioned ingredients in a therapeutically effective dose as referred to elsewhere herein and, preferably, at least one pharmaceutically acceptable carrier and/or diluent. The medicament can be formulated for various routes of administrations set forth elsewhere herein in detail. A therapeutically effective dose refers to amounts of the ingredients to be used for a medicament for treating and/or preventing lymphedema. Therapeutic efficacy and toxicity of the compound can be determined by standard pharmaceutical procedures in cell cultures or experimental animals, e.g., ED50 (the dose therapeutically effective in 50% of the population) and LD50 (the dose lethal to 50% of the population). The dose ratio between therapeutic and toxic effects is the therapeutic index, and it can be expressed as the ratio, LD50/ED50. The dosage regimen will be determined by the attending physician and other clinical factors. As is well known in the medical arts, dosages for any one patient depends upon many factors, including the patient's size, body surface area, age, the particular compound to be administered, sex, time and route of administration, general health, and other drugs being administered concurrently. Progress can be monitored by periodic assessment. The specific dosages referred to in this specification are calculated for subject of mid-age and average weight (about 70 kg).

Specific medicaments based on the composition are prepared in a manner well known in the pharmaceutical art and comprise the active ingredients referred to herein above in admixture or otherwise associated with at least one pharmaceutically acceptable carrier or diluent. For making those specific medicaments, the active ingredients will usually be mixed and, optionally, combined with a carrier or the diluent. The resulting formulations are to be adapted to the mode of administration. The procedures for formulating a medicament as referred to herein may involve mixing, granulating, compression, or dissolving the ingredients as appropriate to form the desired composition. It will be appreciated that the form and character of the pharmaceutical acceptable carrier or diluent is dictated by the amount of active ingredient with which it is to be combined, the route of administration, and other well-known variables. Dosage recommendations shall be indicated in the prescribers or users instructions in order to anticipate dose adjustments depending on the considered recipient. Finally, it is to be understood that the formulation of a composition as a medicament takes place under GMP standardized conditions or the like in order to ensure quality, pharmaceutical security, and effectiveness of the medicament.

Described is a method for the manufacture of a medicament comprising the steps of:
(a) preparing a composition,
   wherein said composition comprises:
   i) Aranea diadematus D6 dilution;
   ii) Calcium phosphoricum D12 dilution;
   iii) Equisetum hiemale D4 ex herba re dilution;
   iv) Ferrum jodatum D12 dilution;
   v) Fumaria officinalis D4 dilution;
   vi) Gentiana lutea D5 dilution;
   vii) Geranium robertianum D4 dilution;
   viii) Levothyroxinum D12 dilution;
   ix) Myosotis arvensis D3 dilution;
   x) Nasturtium officinale D4 dilution;
   xi) Natrium sulfuricum D4 dilution;
   xii) Pinus silvestris D4 dilution;
   xiii) Sarsaparillae radix D6 dilution;
   xiv) Scrophularia nodosa D3 dilution;
   xv) Teucrium scorodonia D3 dilution; and
   xvi) Veronica officinalis D3 dilution,
   and
   wherein said composition to be administered provides the composition in an amount corresponding to the amount provided by at least about 0.125 to 0.25 ml per kg body weight of the subject to be treated every other day; and
(b) formulating said composition as a medicament in a pharmaceutically acceptable manner.

How to prepare the aforementioned components has been set forth elsewhere herein in detail. Moreover, it has been described already how the composition may be formulated as medicament in order to provide the envisaged dosage above.

Finally, described is a method for treating lymphedema in a subject suffering therefrom, said method comprises administering in a therapeutically effective amount to the said subject a composition,
wherein said composition comprises:
i) Aranea diadematus D6 dilution;
ii) Calcium phosphoricum D12 dilution;
iii) Equisetum hiemale D4 ex herba re dilution;
iv) Ferrum jodatum D12 dilution;
v) Fumaria officinalis D4 dilution;
vi) Gentiana lutea D5 dilution;
vii) Geranium robertianum D4 dilution;
viii) Levothyroxinum D12 dilution;
ix) Myosotis arvensis D3 dilution;
x) Nasturtium officinale D4 dilution;
xi) Natrium sulfuricum D4 dilution;
xii) Pinus silvestris D4 dilution;
xiii) Sarsaparillae radix D6 dilution;
xiv) Scrophularia nodosa D3 dilution;
xv) Teucrium scorodonia D3 dilution; and
xvi) Veronica officinalis D3 dilution,
and
wherein said composition to be administered provides the composition in an amount corresponding to the amount provided by at least about 0.125 to 0.25 ml per kg body weight of the subject to be treated every other day.

How to administer the composition referred to above has been described elsewhere herein already in detail. Moreover, the skilled person knows how a therapeutically effective amount can be provided in a suitable form for the present method. Details thereon are found elsewhere in this specification.

### FIGURES

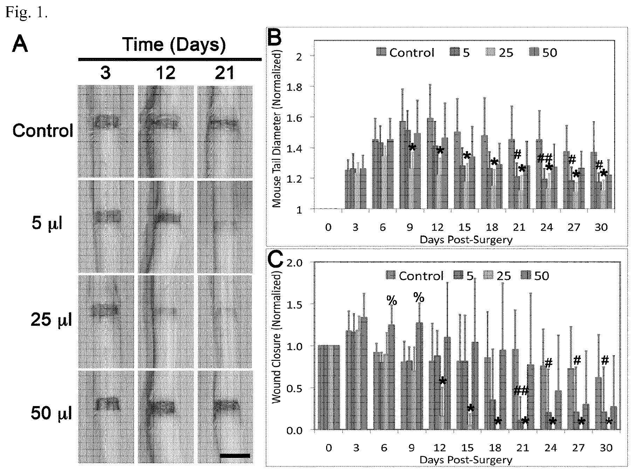

**Figure 1** shows a reduction of tissue swelling and increased wound healing in mice treated with a composition according to the invention (Lymphomyosot N®). Lymphedema in the mouse tail skin was induced over a period of 30 days by a 1 mm wide surgical excision of the skin that was left unprotected. Injury sites at days 3, 12, and 21 for control, 5 µl, 25 µl, and 50 µl drug injection doses are shown from top to bottom, respectively (A). Scale bar in lower right panel = 5 mm. The evolution of tail swelling (B) and wound closure (C) over the 30 day period for the different conditions is graphically depicted. For the swelling data (lymphedema formation), tail diameters are normalized to the average initial (pre-swelling) diameter of the tails within each group. For the wound closure data, wound lengths are normalized to the initial 1 mm long wound. n=10 per group. High statistical significance of the 25 µl drug injection group relative to the control group (p<0.005) is represented by *. Statistical difference between the 5 µl and control group is represented by # (p<0.05) and ## (p<0.005). Statistical difference between the 50 µl and control group is represented by % (p<0.05).

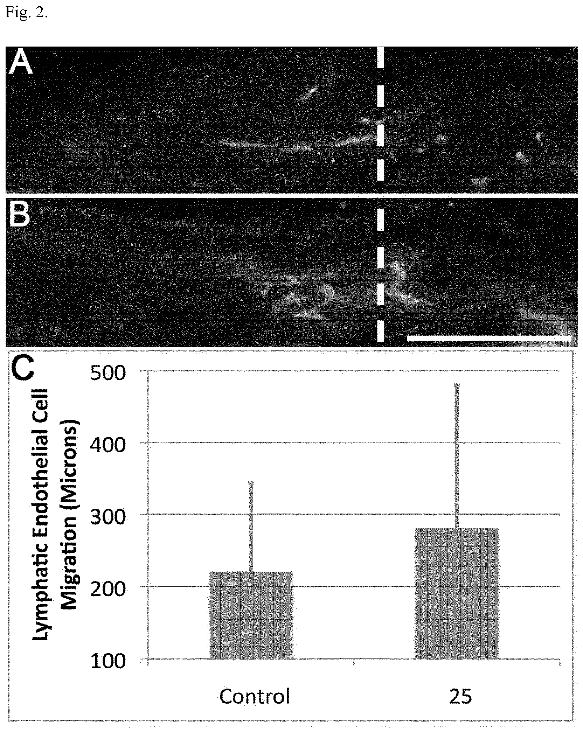

**Figure 2** shows that a composition according to the invention (Lymphomyosot N®) does not increase lymphangiogenesis of lymphatic capillaries. Lymphedema in the mouse tail skin was induced by a 1 mm wide surgical excision of the skin that was protected with a silicone cuff. Images of LYVE-1 labeled (grey color) thick cryo-sections from 10 day post-surgery tail skin are shown for mice treated with 25 µls of saline (A) or drug (B). Scale bar in lower right panel = 0.5 mm. The vertical white dashed line in A and B marks the distal border between native and regenerating tissue. The graph shows distance of lymphatic capillary migration into the regenerating region in the saline and drug treated groups (C). There were no statistical differences between these groups. n=10.

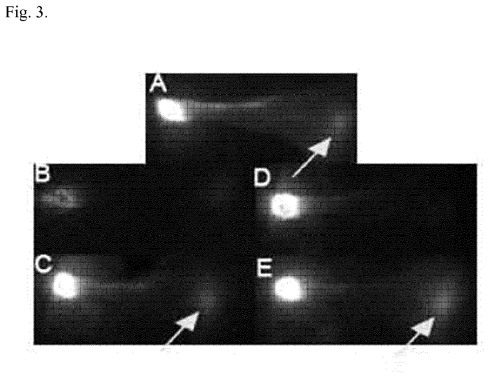

**Figure 3** shows that a composition according to the invention (Lymphomyosot N®) does not increase lymphangiogenesis of lymphatic vessels. Axillary lymph nodes were removed from the mouse and lymphatic vessels in the foreleg were visualized via indocyanine green lymphangiography (grey). Shown are an unoperated control foreleg (A), operated mouse forelegs that received 25 µls of saline at day 10 (B) and day 15 (C), and operated mouse forelegs that received 25 µls of drug at day 10 (D) and day 15 (E). Grey arrow identifies fluorescence signal at the axilla. n=5 per group.

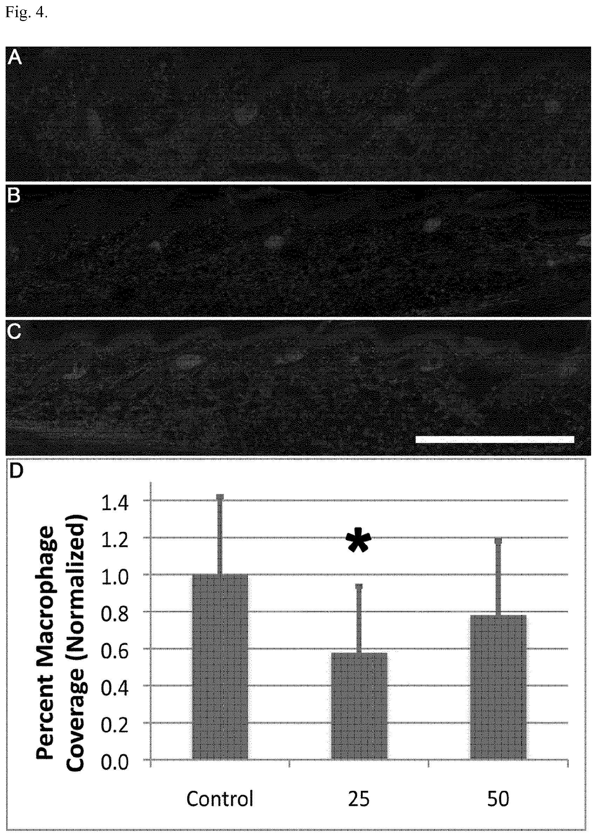

**Figure 4** shows that a composition according to the invention (Lymphomyosot N®) modulates macrophage infiltration during experimental wound healing. Lymphedema in the mouse tail skin was induced by a 1 mm wide surgical excision of the skin that was placed 10 mm from the tail base and left unprotected. Shown are fluorescence images of the F4/80 macrophage marker (A-C) (immune-detected antigen in each image is shown in grey). Macrophages were detected in tissue sections of lymph edematous mouse tail skin treated with saline (A), 25 µls of the drug (B), or 50 µls of the drug (C). The skin epidermis is located at the top of each image. Scale bar in lower right panel of C = 1 mm. Wound margin is to the left of each image (not shown). Distal direction is to the right for each image. Grey color in each image is DAPI labeled cell nuclei. Measurement of macrophage percent coverage for the different treatments (D) demonstrated inhibition of macrophage recruitment in the swollen tissue by the drug treatment. n=10 per group. A significant difference relative to the control condition is indicated by * (p<0.05).

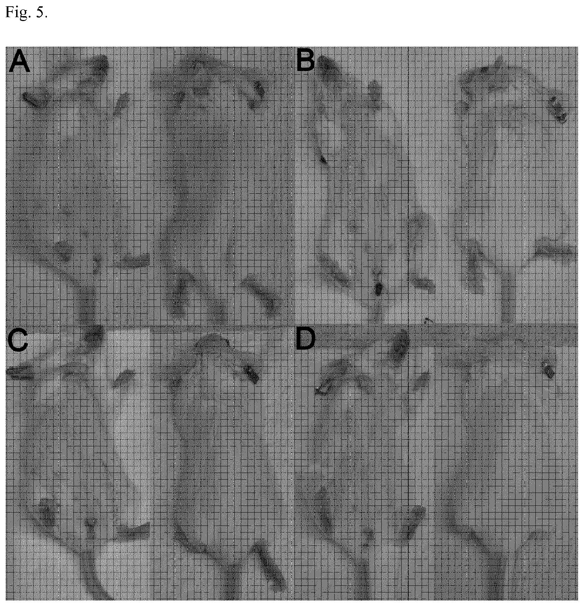

**Figure 5** shows images of mice in supine and prone positions that were collected immediately following euthanization at day 30 in mice that had undergone surgery to as per model 1. Shown are mice that had received 50 µl of saline (A), and mice that had received 5 µl (B), 25 µl (C), or 50 µl (D) of Lymphomyosot N® injections. A yellowish (grey) color is present in the fur of mice receiving 5 µls of the drug (B), but the discoloration was most pronounced in mice receiving the 25 µl drug dose. Discoloration was not evident in the fur of mice receiving the 50 µl dose. n = 10 per group.

### EXAMPLES

### Example 1: General Methods and materials

Female balb/c mice (Jackson Labs; 6-8 weeks) were used. Mice were anesthetized with 2.5% isoflurane mixed with oxygen gas for surgical procedures and were killed at experimental endpoints by CO₂ asphyxiation. All protocols were approved by the Animal Care and Use Committee of Michigan Technological University.

### Model 1 - Tail lymphedema: normal wound healing:

Tail skin edema was created in balb/c mice by excising a 1-mm circular band of dermis (which contained the lymphatic capillary network) 1 cm from the base of the tail, leaving the underlying bone, muscle, tendons, and major blood vessels intact, similar to previous studies (Rutkowski 2006, Microvasc Res 72:161-71; Swartz 1999, J Biomech 32:1297-307) Mice were divided into four groups (ten mice per group) for tail swelling and wound closure measurements and received 5, 25, or 50 microliter i.p. injections of Lymphomyosot N® or 50 microliters of saline on the day of surgery and then every other day until euthanization at day 30. A separate group of mice were divided into three groups (ten mice per group) and received 25 or 50 microliter i.p. injections of Lymphomyosot N® or 50 microliters of saline on the day of surgery and then every other day until euthanization at day 9 for analysis of macrophage recruitment into the swollen tail skin.

### Model 2 - Tail lymphedema: covered wound healing:

A scar-free / enhanced tissue repair model of tail skin edema was created in balb/c mice by removal of dermis as described above, with the regenerating region placed midway down the tail. Following the injury, the wound region was covered with a close-fitting, gas-permeable silicone sleeve (similar to previous studies Ongstad 2010, Am J Physiol Heart Circ Physiol 299:46-54; Boardman 2003, Circ Res 92:801-8; Goldman 2007, AJP 292:2176-83; Goldman 2005, Circ Res 96:1193-9; Goldman 2007, FASEB J 21:1003-12; Pytowski 2005, J Natl Cancer Inst 97:14-21; Rutkowski 2006, Am J Physiol Heart Circ Physiol 291:H1402-10) the sleeve protected the injury site, allowing the tissue to repair more rapidly and in a relatively scar-free manner. Placement of a covered circular wound midway down the tail did not result in edema of the distal tail skin. Mice were divided into two groups (ten mice per group) and received 25 microliters i.p. injections of Lymphomyosot N® or saline on the day of surgery and then every other day until euthanization at day 10 for analysis of lymphatic capillary migration.

### Model 3 - Foreleg lymphedema:

A murine foreleg model of lymphedema was produced as previously described Tammela 2007, Nat Med 13:1458-66). Balb/c mice were anesthetized with 2.5% isoflurane mixed with oxygen gas. Mouse right forepaws were injected subdermally with 1% Trypan Blue for axillary lymph node detection. A 5 mm long surgical incision through the dermis was placed across the axilla on the right side and the axillary lymph nodes were identified and excised along with visible portions of pre and post-nodal collecting vessels under an Olympus SZX7 stereo microscope. The dermis was sutured with 6-0 suture thread (Harvard Apparatus, Holliston, MA) and mice were allowed to recover to day 10 or 15. Mice were divided into two groups (10 mice per group) and received 25 microliters i.p. injections of Lymphomyosot N® or saline on the day of surgery and then every other day until euthanization at days 10 (5 mice per group) and 15 (5 mice per group) for IC-Green imaging and analysis of lymphatic vessel regeneration.

### Immunofluorescence and immunohistochemistry

Tail specimens from model 2 were cut into 10 or 150 µm longitudinal cryosections and immunostained. Immunostaining of tail cryosections proceeded with antibodies against LYVE-1 in the thick sections in order to detect lymphatic endothelial cells (LECs). A goat polyclonal antibody against the lymphatic-specific hyaluronan receptor LYVE-1 (Upstate, Charlottesville, VA) was used along with an Alexa Fluor 488 donkey anti-goat secondary antibody (Invitrogen). Macrophages were detected in the 10 µm cryosections of mouse tail skin by labeling the F4/80 macrophage-specific marker Austyn 1981, Eur J Immunol 11:805-15). A biotinylated anti F4/80 antibody (Serotec) was used along with Alexa Fluor 555 conjugated streptavidin (Invitrogen). Cell nuclei in LYVE-1 and F4/80 labeled sections were counterstained with 4',6-diamino-2-phenylindole (DAPI; Vector Laboratories, Burlingame, CA).

### Physiological measurements

Mouse tail images from the uncovered regenerating skin lymphedema model were captured every three days for each mouse tail with a DP71 color camera mounted to a stereo microscope. Tail swelling was determined with Metamorph software by measuring tail diameters from digital images of the swollen tail distal to the wound site. Wound closure was measured along the longitudinal axis of the tail as the distance between the distal and proximal regenerating tissue across the wound site. Macrophage percent coverage was determined within the swollen skin distal to the wound in F4/80-labeled tissue sections with a threshold analysis using Metamorph software.

### Imaging of functional lymphatic vessels via IC- Green fluorescence lymphography

An imaging system recently developed by Eva M. Sevick-Muraca was used (Kwon 2007 Lymphat Res Biol 5:219-31; Sevick-Muraca 2008, Radiology 246:734-41; Sharma 2007, Am J Physiol Heart Circ Physiol 292:H3109-18) to detect functional lymphatic vessels and lymph nodes in the mouse foreleg. 5 microliters of a 5 mg/ml solution of the fluorescent near-infrared dye Indocyanine Green (ICG; Akorn) was injected into the mouse paw. Detection of ICG was performed with an electron multiplying charge-coupled device (CCD) (Hamamatsu, C9100-13). ICG was illuminated with an array of 760 nm LEDs (Epitex Inc) placed before a 760 nm band pass filter (model 760FS10-50, Andover Corporation) was used to provide the excitation light for activating ICG. A 785 nm and 763 nm custom made holographic notch band rejection filter (model HNPF-785.0-2.0 and HNPF-763.0-2.0, Kaiser Optical Systems) and a 830 nm image quality bandpass filter (model 830FS20-25, Andover Corporation) were placed before the camera lens to selectively reject the excitation light and pass the emitted 830 nm wavelength.

### Statistical Methods

At least five animals were used for each data point. Ten animals were used for most data points. Data are presented as means with standard errors (SE). P values were calculated using ANOVA or t-Test, as indicated.

### Example 2: Reduced tissue swelling by Lymphomyosot N® administration

The ability of Lymphomyosot N® to reduce tissue swelling during lymphedema and to influence wound healing was assessed by employing an experimental model of lymphedema. Wounds and lymphedema were induced in mouse tails as per model 1 and tissue swelling was measured over a 30 day period in mice that received either 5, 25, or 50 microliters of Lymphomyosot N® or 50 microliters of saline, administered via i.p. injections (Figure 1A & 1B). A reduction in tail swelling was found in mice that had received 25 microliters of the drug relative to the saline control group. Differences were highly significant at day 9 and remained highly significant for the duration of the study (p<0.005 by ANOVA). Further found was a reduction in tail swelling during days 21 - 30 in mice that had received 5 microliters of the drug. This difference was highly significant at day 24 (p<0.005 by ANOVA) and significant at the other days (p<0.05 by ANOVA). Differences in tail diameter between the lymph edematous mice receiving 50 microliters of the drug relative to saline administration were not significant at any time. These results show that Lymphomyosot N® reduces swelling and facilitates wound healing of the mouse tail and that 25 microliters of Lymphomyosot N® was the most effective dose for reducing tail diameter, followed by the 5 microliter dose, with no effect from the 50 microliter dose.

### Example 3: Reduced tissue swelling coincides with improved wound repair

Recently, it was shown that tissue swelling in the mouse tail model of lymphedema was worsened when repair of the surgical wound was inhibited by combined neutralization of vascular endothelial growth factor receptor (VEGFR)-2 and VEGFR-3 signaling. In contrast, the swelling was improved when wound repair was augmented with a protective silicone cuff. Inhibition of lymphatic regeneration by neutralizing either VEGFR-2 or VEGFR-3 signaling did not impair resolution of the tail swelling (Ongstad 2010, Am J Physiol Heart Circ Physiol 299:46-54). These results suggested that tissue swelling may be related to wound repair of the lymphatic-related injury rather than, or in addition to, lymphangiogenesis across the wound site. Because in this study a rapid reduction in the tissue swelling in Lymphomyosot N® -treated tails was found, the surgical obstruction for progression of wound repair was examined to determine whether wound repair was related to the tissue swelling. To this end, wound closure between the different groups was quantified and compared. It was found that administration of 25 microliters of Lymphomyosot N® accelerated wound closure relative to treatment with saline (Figure 1C). Differences between these groups were highly significant at day 12 and remained highly significant for the duration of the study (p<0.005, by ANOVA). Administration of 5 microliters of Lymphomyosot N® was also found to accelerate wound closure relative to control wounds (Figure 1C). These differences were highly significant at day 21 (p<0.005, by ANOVA) and significant at days 24 - 30 (p<0.05, by ANOVA). Thus, improved wound repair (Figure 1C) and reduced tail diameter (Figure 1B) by Lymphomyosot N® appeared to be temporally coincident. This finding confirms that Lymphomyosot N® may reduce tissue swelling by increasing wound repair of the surgical obstruction.

### Example 4: Lymphomyosot N® does not affect lymphatic capillary or lymphatic vessel regeneration

Previously it was found that improved repair of the surgical obstruction promotes functional lymphangiogenesis indirectly, as interstitial fluid and lymphatic cells are able to spread freely through a restored, relatively scar-free, matrix bridge (Ongstad 2010, Am J Physiol Heart Circ Physiol 299:46-54; Uzarski 2008, Am J Physiol Heart Circ Physiol 294:1326-34; Goldman 2007, AJP 292:2176-83). In order to determine if Lymphomyosot N® has a direct effect on lymphangiogenesis, a clearly defined region of scar-free regenerating skin within which capillary lymphangiogenesis occurs (as per model 2) was produced. In this model, both the Lymphomyosot N® -treated and the saline-treated control skin experience an augmented wound repair due to protection of the wound site with a silicone cuff. Thus, following drug treatment, any differences obtained in lymphatic cell migration across the site of surgical obstruction would be due to a direct action of the drug rather than an indirect action secondary to improved wound repair. From a previous study it was known that lymphatic migration into the protected regenerating region is detectable in immunolabeled cross sections by post-surgery day 10 (Goldman 2007, FASEB J 21:1003-12). In order to determine the ability of Lymphomyosot N® to increase lymphatic capillary regeneration, groups of mice with covered regenerating regions as per model 2 and administered either 25 microliters of drug or saline via ip injections were prepared. After animal euthanization at day 10, regenerating regions were cryo-sectioned into thick sections for LYVE-1 immunolabeling of lymphatic endothelial cells and 3-dimensional fluorescence microscopy (Figure 2). The distance of lymphatic endothelial cell migration into the regenerating region was measured from LYVE-1 labelled sections for all tails (Figure 2C). Comparisons between groups showed that Lymphomyosot N® did not increase the distance of lymphatic endothelial cell migration (p>0.05 by students t-Test).

Because lymphedema in humans is caused primarily by disruption of lymphatic vessels rather than by disruption of lymphatic capillaries, it was aimed to determine whether Lymphomyosot N® may act directly on regenerating lymphatic vessels. To clarify the ability of Lymphomyosot N® to increase lymphatic vessel regeneration, axillary lymph nodes as per model 3 were removed. Mice underwent surgery for axillary lymph node dissection and received either 25 microliters of drug or saline via ip injections until live animal lymphatic imaging at day 10 or 15 with IC-Green dye (5 mice per group). 10 minutes following an injection of IC-Green into the un-operated mouse foreleg, fluorescent lymph tracer can be seen flowing from the injection site at the paw, through a single lymphatic vessel in the foreleg, and reaching the site of the axillary lymph node (Figure 3A - grey arrow identifies fluorescence signal at axilla), signifying functional transport of the fluorescent dye from the paw injection site to the axilla. Following excision of the axillary lymph nodes in the mouse foreleg, it was found a lack of dye accumulation at the axilla in both drug and saline treated mice at day 10, indicative of poorly functioning lymphatic vessels in both groups of mice at this time (Figure 3B &3D). In contrast, IC-Green dye accumulation was recovered at the site of the axilla by day 15 in both groups (Figure 3C & 3E) indicative of a regenerated lymphatic vessel transport system in the mouse foreleg. The data indicate that Lymphomyosot N® may not influence lymph drainage by regeneration of lymphatic vessels.

### Example 6: Lymphomyosot N® modulates the macrophage infiltration in the swollen tissue

Because Lymphomyosot N® had no affect on regenerating lymphatic capillaries or vessels, it was wondered if the drug may reduce tissue swelling by acting on inflammatory pathways, in addition to improving wound repair. Because it was seen a significant reduction in swelling by day 9 in the 25 microliter drug-treated group, lymphedema was induced in the mouse tail (as per model 1) and tails were assessed at day 9 for macrophage recruitment. Mice received 25 or 50 microliters i.p. injections of Lymphomyosot N® or 50 microliters of saline (10 mice per group). Collected tails were cryo-sectioned and immunolabeled against the F4/80 macrophage maker (Figure 2). Macrophage coverage was found to be highest in the saline control group and significantly reduced in the 25 microliter drug-treated group (p<0.05, by ANOVA; Figure 4D). There was no reduction in the 50 microliter drug-treated group. Lymphomyosot may have accelerated migration of the macrophages out of the affected tissue by the time the tissue was measured and/or modulated their recruitment or inhibited their recruitment.

### Example 7: Yellowish fur discoloration found in Lymphomyosot N® treated mice

During the course of the experimentation to produce the data for Figure 1, a striking yellowish fur discoloration became apparent on the mice receiving the 25 microliters injections of Lymphomyosot N® (Figure 5C). As the discoloration in this group became darker over time, a yellowish discoloration in the fur of mice receiving the 5 microliters injections of Lymphomyosot N® also became apparent (Figure 5B). Mice receiving control saline injections (Figure 5A) or 50 microliters injections of Lymphomyosot N® (Figure 5D) did not become discoloured to the same extent. The origin of the discoloration was not clear, but the yellowish color initiated at the genitalia and spread outwardly over time to discolor the fur at more distant sites. It is possible that altered urine content derived from the Lymphomyosot N® treatment caused the mouse fur yellow staining.

## Claims

1. A composition for use in treating and/or preventing lymphedema,
wherein said composition comprises:
i) Aranea diadematus D6 dilution;
ii) Calcium phosphoricum D 12 dilution;
iii) Equisetum hiemale D4 ex herba re dilution;
iv) Ferrum jodatum D12 dilution;
v) Fumaria officinalis D4 dilution;
vi) Gentiana lutea D5 dilution;
vii) Geranium robertianum D4 dilution;
viii) Levothyroxinum D 12 dilution;
ix) Myosotis arvensis D3 dilution;
x) Nasturtium officinale D4 dilution;
xi) Natrium sulfuricum D4 dilution;
xii) Pinus silvestris D4 dilution;
xiii) Sarsaparillae radix D6 dilution;
xiv) Scrophularia nodosa D3 dilution;
xv) Teucrium scorodonia D3 dilution; and
xvi) Veronica officinalis D3 dilution,
and
wherein said composition is administered in an amount of 0.25 ml per kg body weight of the subject to be treated every other day.

2. The composition for use of claim 1, wherein said composition reduces swelling in lymphedema by supporting the tissue regeneration process and therefore the functional lymphatic regeneration and/or by accelerating/supporting the resolution of the inflammatory process.

3. The composition for use of claim 1 or 2, wherein said lymphedema is associated with lymph node dissection or lymphatic vessel injury.

4. The composition for use of any one of claims 1 to 3, wherein said composition comprises at least one further pharmaceutically active ingredient from Juglans regia.

5. The composition for use of any one of claims 1 to 4, wherein said composition further comprises a pharmaceutically acceptable carrier and/or diluent.

## Patentansprüche

1. Zusammensetzung zur Verwendung bei der Behandlung und/oder der Verhinderung von einem Lymphödem,
wobei die Zusammensetzung umfasst:
i) Aranea diadematus, D6-Verdünnung;
ii) Calcium phosphoricum, D12-Verdünnung;
iii) Equisetum hiemale ex herba re, D4-Verdünnung;
iv) Ferrum jodatum, D12-Verdünnung;
v) Fumaria officinalis, D4-Verdünnung;
vi) Gentiana lutea, D5-Verdünnung;
vii) Geranium robertianum, D4-Verdünnung;
viii)Levothyroxinum, D12-Verdünnung;
ix) Myosotis arvensis, D3-Verdünnung;
x) Nasturtium officinale, D4-Verdünnung;
xi) Natrium sulfuricum, D4-Verdünnung;
xii) Pinus silvestris, D4-Verdünnung;
xiii)Sarsaparillae radix, D6-Verdünnung;
xiv) Scrophularia nodosa, D3-Verdünnung;
xv) Teucrium scorodonia, D3-Verdünnung; und
xvi) Veronica officinalis, D3-Verdünnung,
und
wobei die Zusammensetzung in einer Menge von 0,25 ml pro kg Körpergewicht des zu behandelnden Patienten jeden zweiten Tag verabreicht wird.

2. Zusammensetzung zur Verwendung nach Anspruch 1,
wobei die Zusammensetzung die Schwellung im Lymphödem durch die Unterstützung des Geweberegenerationsprozesses und demzufolge die funktionelle lymphatische Regeneration und/oder durch Beschleunigung/Unterstützung des Rückgangs des Entzündungsprozesses verringert.

3. Zusammensetzung zur Verwendung nach Anspruch 1 oder 2, wobei das Lymphödem mit Lymphknotenentfernung oder Lymphgefäßverletzung verbunden ist.

4. Zusammensetzung zur Verwendung nach einem der Ansprüche 1 bis 3, wobei die Zusammensetzung mindestens einen weiteren pharmazeutischen Wirkstoff aus Juglans regia umfasst.

5. Zusammensetzung zur Verwendung nach einem der Ansprüche 1 bis 4, wobei die Zusammensetzung ferner einen pharmazeutisch annehmbaren Träger und/oder ein pharmazeutisch annehmbares Verdünnungsmittel umfasst.

## Revendications

1. Composition pour utilisation dans le traitement prophylactique et/ou thérapeutique d'un lymphoedème, où ladite composition comprend :
i) Aranea diadematus dilution D6 ;
ii) Calcium phosphoricum dilution D12 ;
iii) Equisetum hiemale redilution D4 ex herba ;
iv) Ferrum jodatum dilution D12 ;
v) Fumaria officinalis dilution D4 ;
vi) Gentiana lutea dilution D5 ;
vii) Geranium robertianum dilution D4 ;
viii) Levothyroxinum dilution D12 ;
ix) Myosotis arvensis dilution D3 ;
x) Nasturtium officinale dilution D4 ;
xi) Natrium sulfuricum dilution D4 ;
xii) Pinus silvestris dilution D4 ;
xiii) Sarsaparillae radix dilution D6 ;
xiv) Scrophularia nodosa dilution D3 ;
xv) Teucrium scorodonia dilution D3 ; et
xvi) Veronica officinalis dilution D3, et
où ladite composition est administrée à une teneur de 0,25 ml par kg de poids corporel du sujet à traiter tous les deux jours.

2. Composition pour utilisation selon la revendication 1, où ladite composition réduit l'oedème du lymphoedème en soutenant le processus de régénération tissulaire et ainsi, la régénération fonctionnelle lymphatique et/ou en accélérant/soutenant la résolution du processus inflammatoire.

3. Composition pour utilisation selon la revendication 1 ou 2, où ledit lymphoedème est associé à une dissection des ganglions lymphatiques ou une lésion des vaisseaux lymphatiques.

4. Composition pour utilisation selon l'une quelconque des revendications 1 à 3, où ladite composition comprend au moins un principe actif pharmaceutique allié issu de Juglans regia.

5. Composition pour utilisation selon l'une quelconque des revendications 1 à 4, où ladite composition comprend en outre un vecteur et/ou diluant pharmaceutiquement acceptable.
